# EUROPEAN PATENT APPLICATION

(11) **EP 4 464 712 A1**
(43) Date of publication of application: **20.11.2024**
(21) Application number: 23740065.0
(22) Date of filing: 12.01.2023
(51) Int. Cl.: C07F 9/6561, A61K 31/675, A61P 11/00, A61P 29/00, A61P 27/02

(54) **CRYSTAL FORM OF NEUROKININ-1 ANTAGONIST PRODRUG COMPOUND**

(30) Priority: 12.01.2022 CN 202210033685
(71) Applicant: Jiangsu Hengrui Pharmaceuticals Co., Ltd., Lianyungang, Jiangsu 222047 (CN)
(72) Inventor: ZHANG, Quanliang, Lianyungang, Jiangsu 222047 (CN); LI, Ming, Lianyungang, Jiangsu 222047 (CN); JIA, Junlei, Lianyungang, Jiangsu 222047 (CN)
(74) Representative: dompatent von Kreisler Selting Werner - Partnerschaft von Patent- und Rechtsanwälten mbB
(86) International application number: PCT/CN2023/071895
(87) International publication number: WO 2023/134723

(57) **Abstract**

The present disclosure relates to a crystal form of a neurokinin-1 antagonist prodrug compound. Specifically, the present disclosure relates to a novel crystal form of a compound represented by formula I and a preparation method. The novel crystal form of the present disclosure has a good physicochemical property and better facilitates clinical treatment.

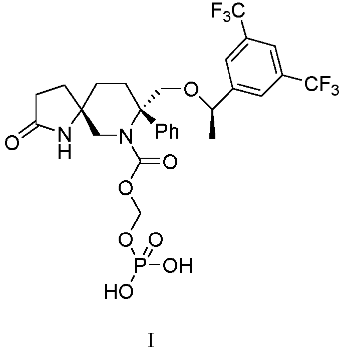

## Description

The present application claims the right of priority for:
CN 202210033685.4, filing date: January 12, 2022.

### TECHNICAL FIELD

The present disclosure relates to a crystal form of a neurokinin-1 antagonist prodrug compound. Specifically, the present disclosure relates to a novel crystal form of a compound represented by formula I and a preparation method therefor.

### BACKGROUND

Tachykinins are peptide ligands for neurokinin receptors. Neurokinin receptors, such as NK1, NK2 and NK3, are involved in various biological processes. They can be found in the nervous and circulatory systems of mammals, as well as in surrounding tissues. Therefore, the regulation of such receptors has been investigated for potential treatment or prevention of various physiological disorders, conditions or diseases in mammals.

On the other hand, drug-induced hemolysis is caused by the destruction of a large number of red blood cells due to immune factors after drugs enter the human body, and clinical signs of hemolysis include anemia, jaundice and soy sauce-colored urine. Drug-induced hemolytic anemia can be divided into the following three types: (1) drug-induced immune hemolytic anemia, which results in an antibody-mediated hemolytic reaction; (2) hemolytic anemia caused by the action of drugs on red blood cells with an inherited enzyme deficiency (e.g., G6PD deficiency); (3) hemolytic anemia caused by a drug-induced hemolytic reaction to abnormal hemoglobin. The key to treating this disease is to discontinue the use of related drugs and control the occurrence of hemolysis so as to prevent complications.

WO 2020259675 provides a new NK1 antagonist prodrug compound (formula I) that is effective in the treatment of various physiological disorders, conditions or diseases with minimal side effects, and that has good pharmaceutical activity.

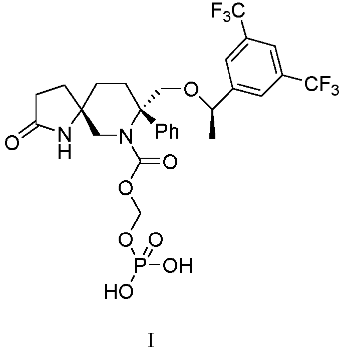

The crystal structure of an active pharmaceutical ingredient often affects the chemical stability of a drug. Different crystallization conditions and storage conditions may lead to changes in the crystal structure of a compound, sometimes accompanied with the production of other crystal forms. In general, an amorphous drug product has an irregular crystal structure, which often leads to other defects, such as poor product stability, finer crystallization, difficulty in filtration, easy to agglomerate, and poor fluidity. Thus, it is necessary to improve various properties of the compound represented by formula I. There is a need for in-depth research to find a novel crystal form with higher crystal purity and good chemical stability.

### CONTENT OF THE PRESENT INVENTION

The present disclosure provides a novel crystal form of a compound represented by formula I, which has good stability and can be better applied in clinical practice.

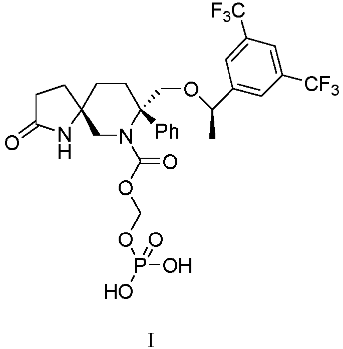

The present disclosure provides crystal form I of a compound represented by formula I, wherein the crystal form I has an X-ray powder diffraction pattern comprising characteristic peaks at 2θ of 4.1, 10.3, 11.6, 12.3, 17.8, 20.6 and 20.8.

The present disclosure provides crystal form I of a compound represented by formula I, wherein the crystal form I has an X-ray powder diffraction pattern comprising characteristic peaks at 2θ of 4.1, 10.3, 10.6, 11.6, 12.3, 13.1, 14.0, 15.0, 16.0, 16.4, 17.1, 17.8, 18.0, 18.3, 18.6, 19.1, 19.4, 19.6, 20.2, 20.6, 20.8, 21.0, 21.7, 22.2, 22.5, 23.0, 23.4, 24.0, 24.3, 24.7, 27.4 and 30.7.

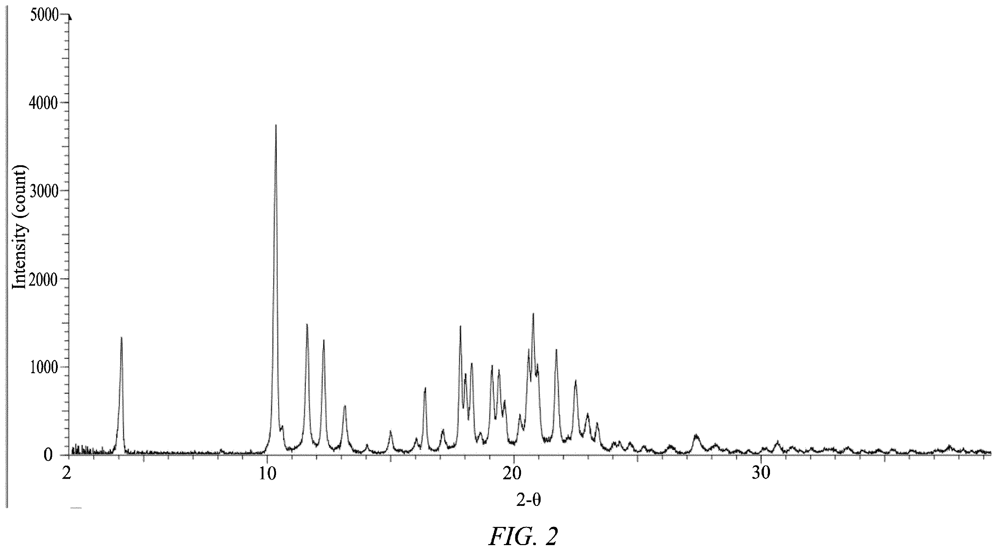

The present disclosure provides crystal form I of a compound represented by formula I, wherein the crystal form I has an X-ray powder diffraction pattern as shown in FIG. 2.

The present disclosure provides crystal form II of a compound represented by formula I, wherein the crystal form II has an X-ray powder diffraction pattern comprising characteristic peaks at 2θ of 5.4, 8.4, 13.6, 17.2, 17.8, 20.6 and 21.5.

The present disclosure provides crystal form II of a compound represented by formula I, wherein the crystal form II has an X-ray powder diffraction pattern comprising characteristic peaks at 2θ of 5.4, 7.8, 8.4, 9.5, 10.7, 11.4, 11.6, 13.6, 13.8, 14.0, 14.5, 14.8, 15.6, 15.8, 16.2, 16.7, 17.2, 17.8, 18.6, 19.0, 19.6, 20.1, 20.6, 21.0, 21.5, 21.8, 22.3, 22.8, 23.3, 23.5, 23.8, 24.1, 24.7, 25.0, 25.4 and 25.7.

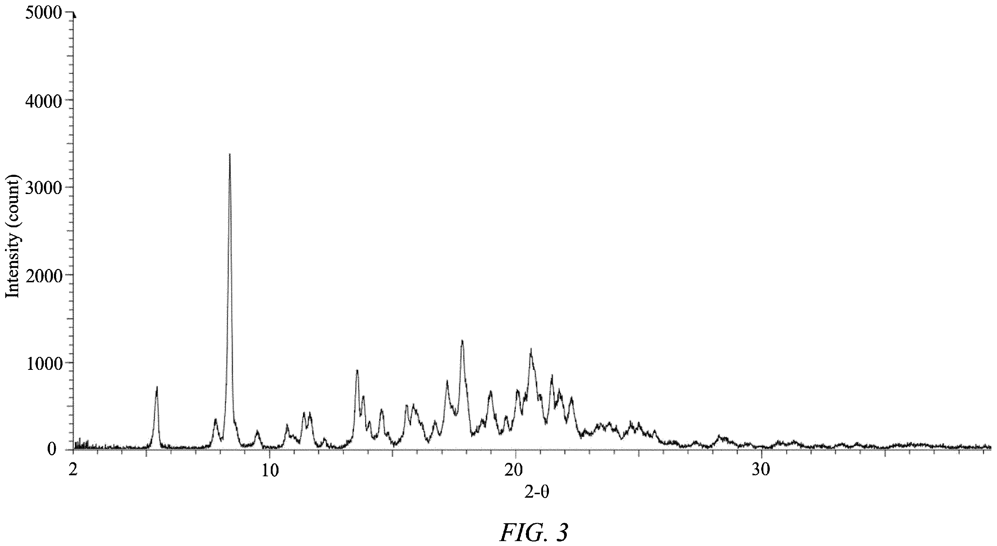

The present disclosure provides crystal form II of a compound represented by formula I, wherein the crystal form II has an X-ray powder diffraction pattern as shown in FIG. 3. The present disclosure provides crystal form III of a compound represented by formula I, wherein the crystal form III has an X-ray powder diffraction pattern comprising characteristic peaks at 2θ of 10.0, 17.6, 18.0, 18.7, 19.5, 20.4 and 20.9.

The present disclosure provides crystal form III of a compound represented by formula I, wherein the crystal form III has an X-ray powder diffraction pattern comprising characteristic peaks at 2θ of 4.8, 10.0, 10.3, 10.6, 11.2, 11.7, 13.4, 14.0, 14.6, 16.5, 17.3, 17.6, 18.0, 18.7, 19.3, 19.5, 20.0, 20.4, 20.9, 21.5, 22.1, 22.4, 22.9, 23.2, 24.6, 25.1, 26.6 and 34.2.

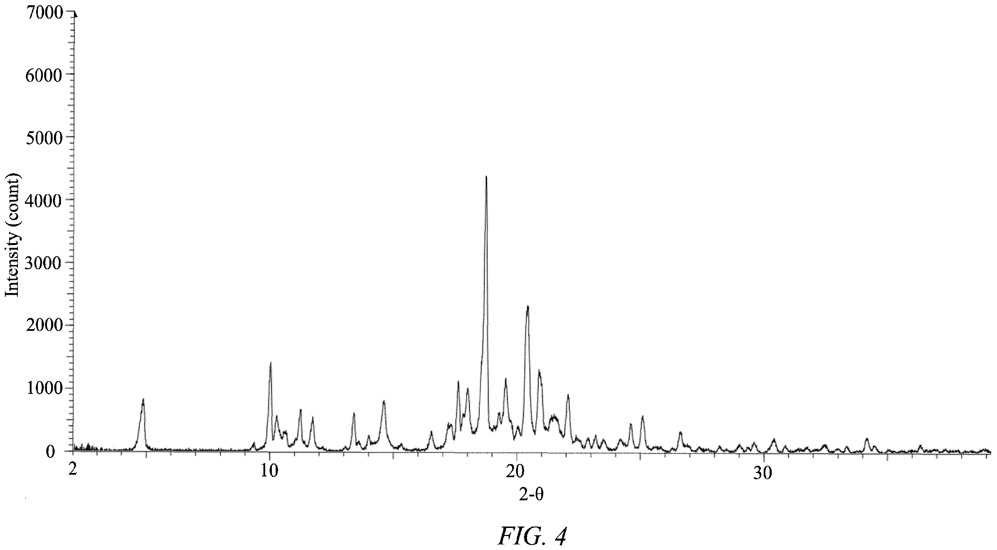

The present disclosure provides crystal form III of a compound represented by formula I, wherein the crystal form III has an X-ray powder diffraction pattern as shown in FIG. 4.

The present disclosure provides a crystal form of a compound represented by formula I, wherein the error range of the 2θ is ± 0.2.

The present disclosure further provides a method for preparing crystal form I of a compound represented by formula I, the method comprising: mixing the compound represented by formula I with an appropriate amount of isopropyl ether under stirring, and separating the solid.

The present disclosure further provides a method for preparing crystal form II of a compound represented by formula I, the method comprising: mixing the compound represented by formula I with an appropriate amount of methyl tert-butyl ether under stirring, and separating the solid.

The present disclosure further provides a method for preparing crystal form III of a compound represented by formula I, the method comprising: mixing the compound represented by formula I with an appropriate amount of 1,4-dioxane under stirring, and separating the solid.

In certain embodiments, the method for preparing the crystal form further comprises a step of adding other solvents for slurrying to remove residual solvents. The other solvents are preferably ethyl acetate, etc. If necessary, slurrying may be carried out at low temperatures, which may be attempted according to the experience of those skilled in the art.

The present disclosure further provides a method for purifying a compound represented by formula I, the method comprising: mixing the compound represented by formula I with methyl tert-butyl ether under stirring, and separating the solid.

In certain embodiments, the method further comprises mixing the separated solid with methyl tert-butyl ether and a second solvent under stirring, precipitating the solid and separating same, wherein the second solvent is selected from one or more of N,N-dimethylformamide, 1-methyl-2-pyrrolidone, dimethyl sulfoxide, tetrahydrofuran, ethyl acetate, isopropyl acetate, butyl acetate, dioxane, toluene, diethyl ether, isopropyl ether, methyl tert-butyl ether, dichloromethane, chloroform, acetone, acetonitrile, methanol, ethanol and isopropanol, preferably ethyl acetate.

Through X-ray powder diffraction pattern (XRPD) and differential scanning calorimetry (DSC), the crystal form obtained in the present disclosure is subjected to structure determination and crystal form study.

The crystallization method of the crystal form in the present disclosure is conventional, such as volatilization crystallization, cooling crystallization, or room-temperature crystallization.

The starting raw materials used in the method for preparing the crystal form in the present disclosure can be any form of the compound represented by formula I, and specific forms include, but are not limited to, amorphous form, any crystal form, hydrate, solvate, etc.

The present disclosure further provides a pharmaceutical composition, comprising the crystal form of the compound represented by formula I, and one or more pharmaceutically acceptable carriers or excipients.

The present disclosure further provides a pharmaceutical composition, which is prepared by using the crystal form of the compound represented by formula I, and one or more pharmaceutically acceptable carriers or excipients.

The present disclosure further provides a method for preparing a pharmaceutical composition, the method comprising a step of mixing the crystal form of the compound represented by formula I with one or more pharmaceutically acceptable carriers or excipients.

The present disclosure further provides the use of the aforementioned crystal form of the compound represented by formula I, or the pharmaceutical composition prepared by the aforementioned method in the manufacture of a medicament for the treatment of a physiological disorder, condition or disease in a patient, wherein the physiological disorder, condition or disease is preferably a respiratory disease, cough, inflammatory disease, skin disorder, ophthalmic disorder, depression, anxiety, phobia, bipolar disorder, alcohol dependence, neuroactive substance abuse, epilepsy, nociception, psychosis, schizophrenia, Alzheimer's disease, AIDS-related dementia, Towne's disease, stress-related disorder, obsessive/compulsive disorder, bulemia, anorexia nervosa, binge eating, mania, premenstrual syndrome, gastrointestinal dysfunction, atherosclerosis, fibrotic disorder, obesity, type II diabetes, headache, neuropathic pain, post-exercise pain, chronic pain syndrome, bladder disorder, urogenital disorder or vomiting or nausea.

The crystal form of the compound represented by formula I of the present disclosure has a good physicochemical property, wherein the crystal form II has lower solvent residue and has obvious advantages as an active pharmaceutical ingredient. Other crystal forms have some residual solvents which are easily dissolved in conventional solvents and are difficult to remove. In addition, active pharmaceutical ingredients with specific crystal forms are more conducive to the manufacture of preparation products due to their excellent physicochemical properties.

In the description and claims of the present application, all scientific and technical terms used herein have the meanings commonly understood by those skilled in the art unless specified otherwise. However, to better understand the present disclosure, definitions and explanations of some related terms are provided below. In addition, when the definitions and explanations of terms provided in the present application have different meanings from those commonly understood by those skilled in the art, the definitions and explanations of terms provided in the present application shall control.

The "X-ray powder diffraction pattern or XRPD" of the present disclosure refers to a set of X-ray powder diffraction patterns determined according to the Bragg's equation 2d sin θ = nλ (in the formula, λ is the wavelength of X-ray, the diffraction order n is any positive integer, and the first-order diffraction peak is generally taken, i.e., n = 1). When X-ray is incident on a certain atomic plane with the d-lattice plane spacing of a crystal or a partial crystal sample at a grazing angle θ (the complement of the angle of incidence, also known as Bragg angle), the Bragg's equation can be satisfied.

The "X-ray powder diffraction pattern or XRPD" of the present disclosure refers to a pattern obtained by using Cu-Kα radiation in an X-ray powder diffractometer.

The "differential scanning calorimetry or DSC" of the present disclosure refers to measuring the temperature difference and heat flow difference between a sample and a reference during the process in which the sample is heated or maintained at a constant temperature, so as to characterize all physical and chemical changes related to thermal effects and obtain the phase change information of the sample.

The "thermogravimetric analysis or TGA" of the present disclosure refers to the continuous measurement of the mass of a sample as a function of temperature or time under programmed temperature control.

The "2θ or angle 2θ" of the present disclosure refers to a diffraction angle, wherein θ is the Bragg angle in ° or degrees, and the error range of the 2θ is ± 0.3, or ± 0.2, or ± 0.1.

The "interplanar spacing or interplanar spacing (d value)" of the present disclosure refers to three non-parallel unit vectors a, b, and c selected from the spatial lattice connecting two adjacent lattice points, by which the lattice is divided into juxtaposed parallelepiped units, called interplanar spacing. The spatial lattice is divided according to the line of the determined parallelepiped units, and a set of straight-line grids are obtained, called spatial lattices or crystal lattices. The lattice and the crystal lattice reflect the periodicity of the crystal structure with geometrical points and lines, respectively. The interplanar spacings (i.e., the distance between two adjacent parallel crystal planes) for different crystal planes are different; the unit is Å or angstrom.

The "mass volume percentage" (w/v) of the present disclosure refers to the mass (in g) of the component contained per 100 mL of a liquid system, i.e., g/100 mL.

### BRIEF DESCRIPTION OF THE DRAWINGS

FIG. 1 shows an XRPD pattern of an amorphous form of the compound represented by formula I;
FIG. 2 shows an XRPD pattern of crystal form I of the compound represented by formula I;
FIG. 3 shows an XRPD pattern of crystal form II of the compound represented by formula I;
FIG. 4 shows an XRPD pattern of crystal form III of the compound represented by formula I.

### DETAILED DESCRIPTION OF THE PREFERRED EMBODIMENT

The present disclosure will be explained in more detail below in conjunction with examples. The examples in the present disclosure are only used to illustrate the technical solutions of the present disclosure and do not limit the essence and scope of the present disclosure.

Test conditions for instruments used in the experiment:
1. Differential Scanning Calorimeter (DSC)
   Instrument model: METTLER TOLEDO DSC 1
   Purge gas: nitrogen
   Heating rate: 10.0 °C/min
   Temperature range: 40-250 °C
2. X-ray Powder Diffraction (XRPD)
   Instrument model: BRUKER D8 FOCUS
   Ray: monochromatic Cu-Kα ray (Cu-Kα1 wavelength being 1.5406Å, Cu-Kα2 wavelength being 1.54439Å, Cu-Kα wavelength taking a weighted average of Kα1 and Kα2 (λ = 1.5418Å))
   Scanning mode: θ/2θ, scanning range: 2-40°
   Voltage: 40 KV, current: 40 mA

The structures of the compounds are determined by nuclear magnetic resonance (NMR) spectroscopy or/and mass spectrometry (MS). The NMR shift (δ) is given in the unit of 10⁻⁶ (ppm). NMR is determined by Bruker AVANCE-400 nuclear magnetic resonance spectrometer; the solvents for determination are deuterated dimethyl sulfoxide (DMSO-*d₆*), deuterated chloroform (CDCl₃) and deuterated methanol (CD₃OD); and the internal standard is tetramethylsilane (TMS).

MS is determined by FINNIGAN LCQAd (ESI) mass spectrometer (manufacturer: Thermo, model: Finnigan LCQ advantage MAX).

High performance liquid chromatography (HPLC) analysis is carried out by Agilent HPLC 1200DAD, Agilent HPLC 1200VWD and Waters HPLC e2695-2489 high-pressure liquid chromatographs.

Chiral HPLC analysis is carried out by Agilent 1260 DAD high-performance liquid chromatograph.

Preparative high performance liquid chromatography is carried out by Waters 2767, Waters 2767-SQ Detecor2, Shimadzu LC-20AP and Gilson-281 preparative chromatographs.

Chiral preparative high performance liquid chromatography is carried out by Shimadzu LC-20AP preparative chromatograph.

The CombiFlash flash preparative instrument used is Combiflash Rf200 (TELEDYNE ISCO).

The silica gel plate for thin layer chromatography is Yantai Huanghai HSGF254 or Qingdao GF254 silica gel plate, the specification of the silica gel plate used for thin layer chromatography (TLC) is 0.15 mm to 0.2 mm, and the specification of the silica gel plate used for separating and purifying products by thin layer chromatography is 0.4 mm to 0.5 mm.

For silica gel column chromatography, Yantai Huanghai silica gel of 200-300 mesh silica gel is generally used as a carrier.

The known starting raw materials of the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

If there is no special instruction in the examples, reactions can be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Parr 3916EKX hydrogenator, Qinglan QL-500 hydrogen generator or HC2-SS hydrogenator are used for pressurized hydrogenation reaction.

The hydrogenation reaction usually comprises the steps of vacuumizing and charging with hydrogen, and the operation is repeated for 3 times.

CEM Discover-S 908860 microwave reactor is used for the microwave reaction.

Unless otherwise indicated, the solution in the examples refers to an aqueous solution.

Unless otherwise indicated, the reaction temperature in the examples refers to room temperature, which is 20 °C to 30 °C.

The progress of the reaction in the examples is monitored by means of thin layer chromatography (TLC). The developing solvent used in the reaction, the eluent system of column chromatography used in the purification of compounds and the developing solvent system of thin layer chromatography include: A: n-hexane/ethyl acetate system, B: dichloromethane/methanol system, wherein the volume ratio of the solvent is adjusted according to the polarity of compounds and can also be adjusted by adding a small amount of basic reagents such as triethylamine or acidic reagents such as acetic acid.

### Example 1: Preparation of compound represented by formula I

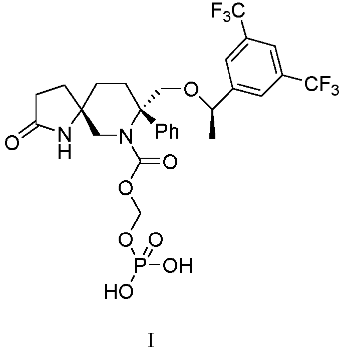

### Step 1:

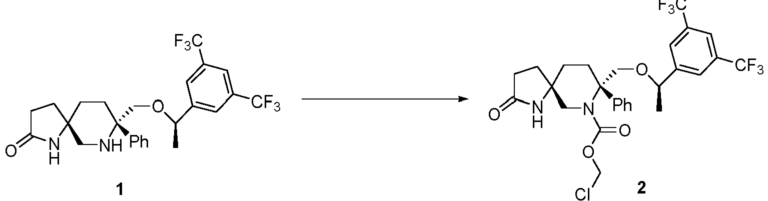

Under N₂ protection, in a 100 mL three-necked flask, compound 1 (2.43 g, 4.86 mmol, 1 eq) was weighed and dissolved in dichloromethane (36 mL), diisopropylethylamine (5 g, 38.76 mmol, 8 eq) was added, and the mixture was cooled to -30 °C. Trimethylchlorosilane (1.36 g, 12.52 mmol, 2.6 eq) was added, and the mixture was stirred at room temperature for 2 h. Then the mixture was cooled to -25 °C, and a solution of chloromethyl chloroformate (0.77 g, 6 mmol, 1.23 eq) in dichloromethane was added dropwise. The resulting mixture was stirred at a temperature controlled at -20 °C to -5 °C until the reaction was completed. The reaction liquid was poured into iced water, and the liquid was separated and extracted with dichloromethane. Water and 1 N hydrogen chloride solution were added, the liquid was separated, and then the resultant was washed successively with sodium chloride aqueous solution, saturated sodium bicarbonate aqueous solution and sodium chloride aqueous solution, dried over anhydrous sodium sulfate, filtered and concentrated to afford a product as a yellow gum (3.0 g, yield: 104%).

### Step 2:

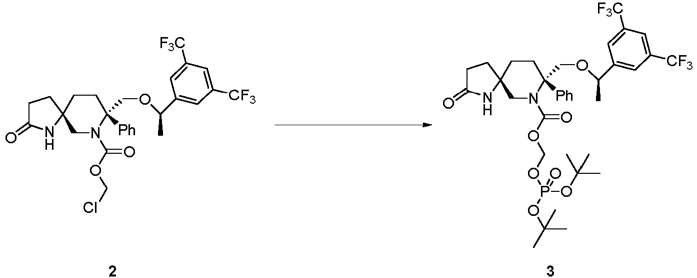

Under N₂ protection, in a 500 mL three-necked flask, compound 2 (2.8 g, 4.53 mmol, 1 eq), tetrabutylammonium iodide (1.68 g, 4.55 mmol, 1 eq), potassium di-tert-butylphosphate (5.63 g, 22.67 mmol, 5 eq) and dioxane (84 mL) were added , and the mixture was heated to 55 °C and stirred for 4 h. The reaction liquid was cooled and poured into ethyl acetate and water. The liquid was separated and extracted with ethyl acetate. The extract was washed with sodium sulfite aqueous solution, then washed successively with water and sodium chloride aqueous solution, dried over anhydrous sodium sulfate, filtered, and concentrated to afford a product as a yellow foam (3.73 g, yield: 107%).

### Step 3:

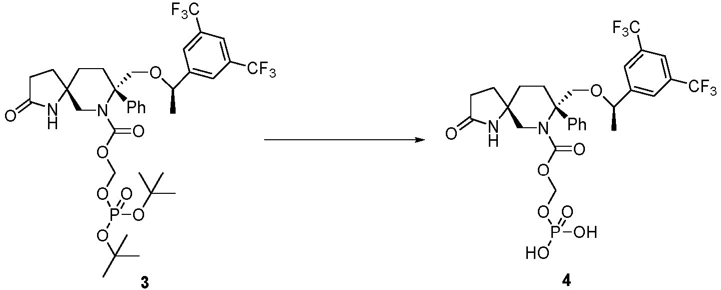

Under N₂ protection, in a 500 mL single-necked flask, compound 3 (6.65 g, 8.67 mmol, 1 eq) was dissolved in dichloromethane (200 mL). Under ice water cooling, trifluoroacetic acid (9.89 g, 86.7 mmol, 10.0 eq) was slowly added. The resulting mixture was stirred until the reaction was completed, and the reaction liquid was concentrated to afford a product as an oil (2.29 g). The oil was purified by reverse phase silica gel column (C18) (solution A: 20 mmol NH₄HCO₃ aqueous solution, solution B: acetonitrile), adjusted to pH = 1-2 with 1 M phosphoric acid, extracted with dichloromethane, washed with saturated sodium chloride aqueous solution, dried over anhydrous sodium sulfate, filtered, and concentrated to afford a target product (2.7 g).

¹H-NMR (400MHz, CD₃OD): δ(ppm) 7.89(s, 2H), 7.86(s, 1H), 7.41-7.27(m, 5H), 5.66(d, J=12Hz, 1H), 5.50-5.47(m, 1H), 4.60(d, J=8Hz, 1H), 4.20-3.88(m, 3H), 2.51-2.10(m, 5H), 1.86-1.66(m, 3H), 1.44-1.31(m, 4H).

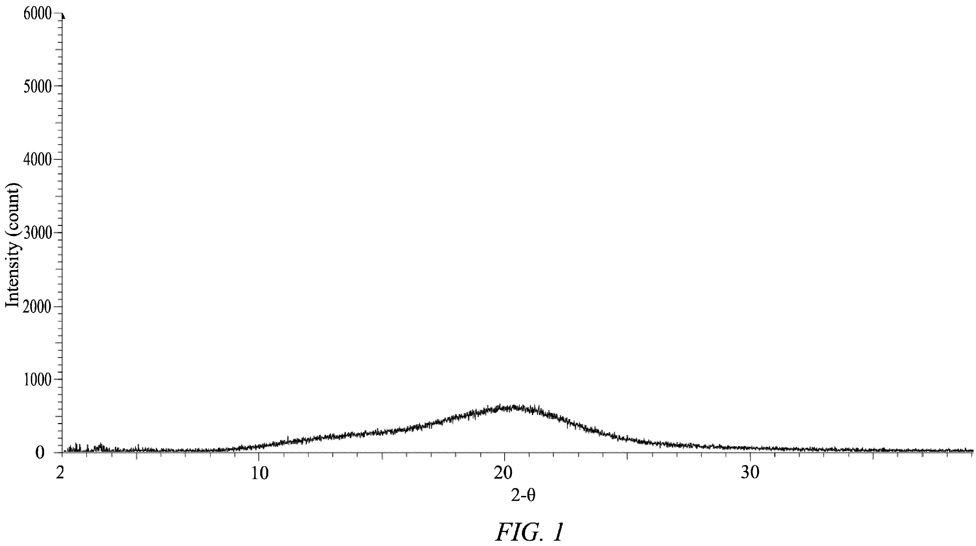

After detection by X-ray powder diffraction, compound 4 (i.e., the compound represented by formula I) is present in an amorphous form, and the XRPD pattern is as shown in FIG. 1.

### Example 2: Preparation of crystal form I of compound represented by formula I

About 5.0 g of the compound represented by formula I was weighed, and 50 ml of isopropyl ether was added. The mixture was stirred until the solid was dissolved, and continuously stirred until the solid was precipitated. The resulting mixture was filtered, and dried under vacuum to afford crystal form I of the compound represented by formula I. The XRPD pattern is as shown in FIG. 2, and the positions of the characteristic peaks thereof are as shown in Table 1. The DSC pattern showed that the peak value of an endothermic peak was 102.48 °C.

**Table 1: XRPD characteristic peak positions of crystal form I**

| Peak no. | 2θ[°] | d[Å] | I[%] |
|---|---|---|---|
| Peak 1 | 4.06 | 21.758 | 35.7 |
| Peak 2 | 8.12 | 10.876 | 1.5 |
| Peak 3 | 10.32 | 8.563 | 100.0 |
| Peak 4 | 10.60 | 8.338 | 8.5 |
| Peak 5 | 11.59 | 7.627 | 39.7 |
| Peak 6 | 12.26 | 7.212 | 34.9 |
| Peak 7 | 13.11 | 6.746 | 16.3 |
| Peak 8 | 14.03 | 6.308 | 3.0 |
| Peak 9 | 14.98 | 5.909 | 7.1 |
| Peak 10 | 15.37 | 5.762 | 1.6 |
| Peak 11 | 16.02 | 5.529 | 4.9 |
| Peak 12 | 16.37 | 5.412 | 19.7 |
| Peak 13 | 17.10 | 5.182 | 7.6 |
| Peak 14 | 17.81 | 4.976 | 39.2 |
| Peak 15 | 18.01 | 4.921 | 24.5 |
| Peak 16 | 18.27 | 4.853 | 27.8 |
| Peak 17 | 18.62 | 4.762 | 6.6 |
| Peak 18 | 19.09 | 4.646 | 27.1 |
| Peak 19 | 19.38 | 4.576 | 25.7 |
| Peak 20 | 19.61 | 4.523 | 16.5 |
| Peak 21 | 20.24 | 4.383 | 12.1 |
| Peak 22 | 20.58 | 4.312 | 32.1 |
| Peak 23 | 20.78 | 4.271 | 43.0 |
| Peak 24 | 20.95 | 4.236 | 27.6 |
| Peak 25 | 21.72 | 4.089 | 32.0 |
| Peak 26 | 22.21 | 3.999 | 5.0 |
| Peak 27 | 22.50 | 3.949 | 22.5 |
| Peak 28 | 22.98 | 3.867 | 12.4 |
| Peak 29 | 23.38 | 3.802 | 9.5 |
| Peak 30 | 24.04 | 3.698 | 3.7 |
| Peak 31 | 24.27 | 3.664 | 4.0 |
| Peak 32 | 24.71 | 3.600 | 3.7 |
| Peak 33 | 25.26 | 3.523 | 2.6 |
| Peak 34 | 25.55 | 3.483 | 1.4 |
| Peak 35 | 26.32 | 3.383 | 2.2 |
| Peak 36 | 27.38 | 3.255 | 6.1 |
| Peak 37 | 28.18 | 3.164 | 2.8 |
| Peak 38 | 28.59 | 3.120 | 1.6 |
| Peak 39 | 29.09 | 3.067 | 1.1 |
| Peak 40 | 29.51 | 3.024 | 1.3 |
| Peak 41 | 30.18 | 2.958 | 1.8 |
| Peak 42 | 30.68 | 2.912 | 4.0 |
| Peak 43 | 31.27 | 2.859 | 2.2 |
| Peak 44 | 31.61 | 2.828 | 1.2 |
| Peak 45 | 32.05 | 2.791 | 1.9 |
| Peak 46 | 32.62 | 2.743 | 1.2 |
| Peak 47 | 32.89 | 2.721 | 1.7 |
| Peak 48 | 33.53 | 2.670 | 2.2 |
| Peak 49 | 34.12 | 2.625 | 1.2 |
| Peak 50 | 34.78 | 2.577 | 1.6 |
| Peak 51 | 35.35 | 2.537 | 1.4 |
| Peak 52 | 36.11 | 2.485 | 1.3 |
| Peak 53 | 37.14 | 2.419 | 1.1 |
| Peak 54 | 37.65 | 2.387 | 2.3 |
| Peak 55 | 38.21 | 2.353 | 1.9 |
| Peak 56 | 38.55 | 2.334 | 1.1 |
| Peak 57 | 38.88 | 2.315 | 1.3 |
| Peak 58 | 39.24 | 2.294 | 0.7 |

### Example 3: Preparation of crystal form II of compound represented by formula I

About 1.0 g of the compound represented by formula I was weighed, and 6 ml of methyl tert-butyl ether was added. The mixture was stirred until the solid was dissolved. The resulting mixture was cooled down for crystallization in an ice-water bath for 2 h and filtered. The filter cake was slurried for 2 h by adding 1350 µl of ethyl acetate and 1350 µl of methyl tert-butyl ether, and then slurried in an ice-water bath for 1 h, filtered, and dried under vacuum to afford crystal form II of the compound represented by formula I. The XRPD pattern is as shown in FIG. 3, and the positions of the characteristic peaks thereof are as shown in Table 2. The DSC pattern showed that the peak value of an endothermic peak was 126.07 °C.

**Table 2: XRPD characteristic peak positions of crystal form II**

| Peak no. | 2θ[°] | d[Å] | I[%] |
|---|---|---|---|
| Peak 1 | 5.38 | 16.419 | 20.0 |
| Peak 2 | 7.79 | 11.344 | 10.2 |
| Peak 3 | 8.36 | 10.570 | 100.0 |
| Peak 4 | 9.48 | 9.319 | 6.4 |
| Peak 5 | 10.70 | 8.260 | 8.0 |
| Peak 6 | 11.38 | 7.772 | 12.4 |
| Peak 7 | 11.63 | 7.603 | 12.2 |
| Peak 8 | 12.21 | 7.241 | 3.7 |
| Peak 9 | 13.55 | 6.529 | 26.6 |
| Peak 10 | 13.79 | 6.418 | 17.8 |
| Peak 11 | 14.04 | 6.302 | 9.4 |
| Peak 12 | 14.54 | 6.085 | 13.5 |
| Peak 13 | 14.80 | 5.980 | 5.7 |
| Peak 14 | 15.56 | 5.692 | 14.8 |
| Peak 15 | 15.84 | 5.590 | 15.2 |
| Peak 16 | 16.18 | 5.473 | 9.1 |
| Peak 17 | 16.71 | 5.300 | 9.6 |
| Peak 18 | 17.21 | 5.148 | 23.3 |
| Peak 19 | 17.83 | 4.971 | 36.6 |
| Peak 20 | 18.64 | 4.756 | 9.5 |
| Peak 21 | 18.98 | 4.673 | 19.7 |
| Peak 22 | 19.61 | 4.524 | 10.9 |
| Peak 23 | 20.09 | 4.417 | 19.9 |
| Peak 24 | 20.62 | 4.303 | 34.1 |
| Peak 25 | 20.99 | 4.229 | 18.5 |
| Peak 26 | 21.47 | 4.135 | 24.0 |
| Peak 27 | 21.79 | 4.075 | 19.6 |
| Peak 28 | 22.26 | 3.991 | 17.2 |
| Peak 29 | 22.84 | 3.891 | 6.1 |
| Peak 30 | 23.29 | 3.816 | 8.1 |
| Peak 31 | 23.47 | 3.787 | 8.6 |
| Peak 32 | 23.79 | 3.737 | 9.1 |
| Peak 33 | 24.07 | 3.695 | 7.2 |
| Peak 34 | 24.69 | 3.603 | 7.7 |
| Peak 35 | 25.02 | 3.557 | 9.0 |
| Peak 36 | 25.35 | 3.510 | 5.8 |
| Peak 37 | 25.66 | 3.469 | 6.5 |
| Peak 38 | 26.48 | 3.363 | 2.6 |
| Peak 39 | 27.32 | 3.261 | 2.4 |
| Peak 40 | 28.29 | 3.152 | 4.2 |
| Peak 41 | 28.55 | 3.124 | 3.8 |
| Peak 42 | 28.81 | 3.096 | 2.8 |
| Peak 43 | 29.53 | 3.022 | 2.1 |
| Peak 44 | 30.69 | 2.911 | 2.8 |
| Peak 45 | 31.35 | 2.851 | 2.4 |
| Peak 46 | 33.28 | 2.690 | 2.0 |
| Peak 47 | 33.88 | 2.644 | 2.2 |
| Peak 48 | 34.55 | 2.594 | 1.0 |
| Peak 49 | 36.11 | 2.486 | 1.8 |
| Peak 50 | 39.09 | 2.302 | 1.5 |

### Example 4: Preparation of crystal form III of compound represented by formula I

About 2.0 g of the compound represented by formula I was weighed, and 30 ml of 1,4-dioxane was added. The mixture was stirred at room temperature for clear dissolution, and continuously stirred until the solid was precipitated. The resulting mixture was filtered, and dried under vacuum to afford crystal form III of the compound represented by formula I. The XRPD pattern is as shown in FIG. 4, and the positions of the characteristic peaks thereof are as shown in Table 3. The DSC pattern showed that the peak value of an endothermic peak was 112.36 °C.

**Table 3: XRPD characteristic peak positions of crystal form III**

| Peak no. | 2θ[°] | d[Å] | I[%] |
|---|---|---|---|
| Peak 1 | 4.82 | 18.333 | 18.8 |
| Peak 2 | 9.32 | 9.481 | 2.7 |
| Peak 3 | 9.99 | 8.851 | 30.4 |
| Peak 4 | 10.26 | 8.613 | 13.0 |
| Peak 5 | 10.60 | 8.340 | 6.9 |
| Peak 6 | 11.22 | 7.881 | 15.3 |
| Peak 7 | 11.70 | 7.555 | 11.1 |
| Peak 8 | 13.04 | 6.781 | 1.9 |
| Peak 9 | 13.38 | 6.612 | 14.0 |
| Peak 10 | 13.61 | 6.502 | 3.3 |
| Peak 11 | 13.99 | 6.325 | 5.7 |
| Peak 12 | 14.60 | 6.062 | 18.6 |
| Peak 13 | 15.30 | 5.787 | 2.9 |
| Peak 14 | 16.53 | 5.359 | 7.4 |
| Peak 15 | 17.30 | 5.123 | 9.3 |
| Peak 16 | 17.61 | 5.032 | 25.7 |
| Peak 17 | 17.99 | 4.927 | 23.2 |
| Peak 18 | 18.72 | 4.737 | 100.0 |
| Peak 19 | 19.28 | 4.601 | 13.8 |
| Peak 20 | 19.53 | 4.541 | 26.7 |
| Peak 21 | 20.04 | 4.427 | 9.2 |
| Peak 22 | 20.41 | 4.348 | 53.1 |
| Peak 23 | 20.92 | 4.243 | 28.9 |
| Peak 24 | 21.51 | 4.127 | 13.5 |
| Peak 25 | 22.07 | 4.025 | 21.0 |
| Peak 26 | 22.43 | 3.961 | 5.2 |
| Peak 27 | 22.88 | 3.884 | 5.3 |
| Peak 28 | 23.19 | 3.833 | 6.2 |
| Peak 29 | 23.52 | 3.780 | 4.9 |
| Peak 30 | 24.20 | 3.674 | 4.8 |
| Peak 31 | 24.63 | 3.611 | 10.2 |
| Peak 32 | 25.11 | 3.544 | 13.4 |
| Peak 33 | 26.31 | 3.384 | 1.9 |
| Peak 34 | 26.63 | 3.345 | 7.6 |
| Peak 35 | 26.91 | 3.311 | 3.2 |
| Peak 36 | 27.40 | 3.252 | 2.1 |
| Peak 37 | 28.22 | 3.159 | 2.6 |
| Peak 38 | 29.02 | 3.074 | 3.2 |
| Peak 39 | 29.37 | 3.038 | 2.4 |
| Peak 40 | 29.63 | 3.013 | 4.0 |
| Peak 41 | 30.42 | 2.936 | 4.9 |
| Peak 42 | 30.89 | 2.892 | 2.9 |
| Peak 43 | 31.75 | 2.816 | 2.0 |
| Peak 44 | 32.49 | 2.753 | 2.9 |
| Peak 45 | 33.01 | 2.711 | 1.3 |
| Peak 46 | 33.38 | 2.683 | 2.5 |
| Peak 47 | 34.20 | 2.620 | 5.5 |
| Peak 48 | 34.51 | 2.597 | 2.6 |
| Peak 49 | 35.10 | 2.554 | 1.4 |
| Peak 50 | 36.37 | 2.468 | 3.0 |
| Peak 51 | 36.95 | 2.431 | 1.7 |
| Peak 52 | 37.38 | 2.404 | 1.8 |
| Peak 53 | 38.96 | 2.310 | 1.4 |
| Peak 54 | 39.79 | 2.264 | 1.0 |

### Example 5: Solvent residue of crystal form of compound represented by formula I

The solvent residue of the crystal form of the compound represented by formula I was determined by gas chromatography, as shown in the table below. Crystal form II has the lowest solvent residue.

**Table 4**

| Sample | 1,4-Dioxane | Methyl tert-butyl ether | Isopropyl ether | Ethyl acetate | Ethylene glycol dimethyl ether |
|---|---|---|---|---|---|
| **Crystal form I** | Not detected | Not detected | 12.0% | 0.0293% | Not detected |
| **Crystal form II** | Not detected | 0.1867% | Not detected | 0.0105% | Not detected |
| **Crystal form III** | 6.3% | Not detected | Not detected | Not detected | Not detected |

### Example 6: Preparation of pharmaceutical composition comprising compound represented by formula I

Tert-butanol and water were mixed at a volume ratio of 1 : 1 to form a 50% tert-butanol solution. 90% of the target volume of the solution was taken, and 5% (mass volume percentage) of mannitol and 4.36% of the raw material of the compound represented by formula I were added. The mixture was stirred at room temperature for dissolution (dissolution being considered as the absence of significant blocky or flocculent insoluble substances in the solution). The pH was adjusted to 5 with dilute hydrochloric acid or sodium hydroxide, filtration was performed with a 45 µm organic filtration membrane to obtain a clear solution, and the volume of the clear solution was made constant with 50% tert-butanol. The above-mentioned solution was stored at 4 °C before subpackaging. After subpackaging according to the target volume, half of a stopper was pressed, and a freeze-drying procedure was run. After the completion of the procedure, nitrogen was filled, the remaining stopper was pressed, and the freeze-dried compounds were taken out of the box.

When the raw material of the compound represented by formula I was in the crystal form II thereof, the raw material dissolved faster, no agglomeration occurred, and there were no significant blocky or flocculent insoluble substances in the solution, without affecting the preparation of the preparation.

When the raw material of the compound represented by formula I was in the amorphous form thereof, the raw material agglomerated in a large amount in the solution, resulting in the inability to completely dissolve the raw material and making it difficult to prepare a lyophilized preparation.

### Example 7

1.0 g of the crystal form I of the compound represented by formula I was added to 1.5 mL of ethyl acetate, and the mixture was stirred at room temperature, filtered, and dried under vacuum. The conversion to the crystal form II of the compound represented by formula I was measured.

### Example 8

1.0 g of the crystal form III of the compound represented by formula I was added to 1.5 mL of ethyl acetate, and the mixture was stirred at room temperature, filtered, and dried under vacuum. The conversion to the crystal form II of the compound represented by formula I was measured.

## Claims

1. Crystal form I of a compound represented by formula I, 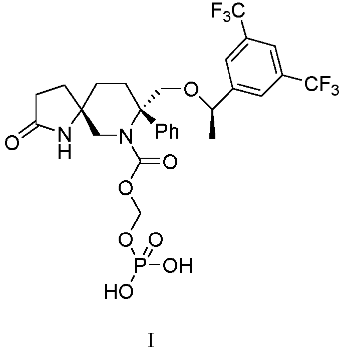 wherein the crystal form I has an X-ray powder diffraction pattern comprising characteristic peaks at 2θ of 4.1, 10.3, 11.6, 12.3, 17.8, 20.6 and 20.8.

2. The crystal form I of the compound represented by formula I according to claim 1, wherein the crystal form I has an X-ray powder diffraction pattern comprising characteristic peaks at 2θ of 4.1, 10.3, 10.6, 11.6, 12.3, 13.1, 14.0, 15.0, 16.0, 16.4, 17.1, 17.8, 18.0, 18.3, 18.6, 19.1, 19.4, 19.6, 20.2, 20.6, 20.8, 21, 21.7, 22.2, 22.5, 23.0, 23.4, 24.0, 24.3, 24.7, 27.4 and 30.7.

3. The crystal form I of the compound represented by formula I according to claim 1, wherein the crystal form I has an X-ray powder diffraction pattern as shown in FIG. 2.

4. Crystal form II of a compound represented by formula I, wherein the crystal form II has an X-ray powder diffraction pattern comprising characteristic peaks at 2θ of 5.4, 8.4, 13.6, 17.2, 17.8, 20.6 and 21.5.

5. The crystal form II of the compound represented by formula I according to claim 4, wherein the crystal form II has an X-ray powder diffraction pattern comprising characteristic peaks at 2θ of 5.4, 7.8, 8.4, 9.5, 10.7, 11.4, 11.6, 13.6, 13.8, 14.0, 14.5, 14.8, 15.6, 15.8, 16.2, 16.7, 17.2, 17.8, 18.6, 19.0, 19.6, 20.1, 20.6, 21.0, 21.5, 21.8, 22.3, 22.8, 23.3, 23.5, 23.8, 24.1, 24.7, 25.0, 25.4 and 25.7.

6. The crystal form II of the compound represented by formula I according to claim 4, wherein the crystal form II has an X-ray powder diffraction pattern as shown in FIG. 3.

7. Crystal form III of a compound represented by formula I, wherein the crystal form III has an X-ray powder diffraction pattern comprising characteristic peaks at 2θ of 10.0, 17.6, 18.0, 18.7, 19.5, 20.4 and 20.9.

8. The crystal form III of the compound represented by formula I according to claim 7, wherein the crystal form III has an X-ray powder diffraction pattern as shown in FIG. 4.

9. The crystal form of the compound represented by formula I according to any one of claims 1 to 8, wherein the error range of the 2θ is ±0.2.

10. A method for preparing the crystal form II of the compound represented by formula I according to any one of claims 4 to 6, the method comprising: mixing the compound represented by formula I with an appropriate amount of methyl tert-butyl ether under stirring, and separating the solid.

11. A pharmaceutical composition, comprising the crystal form of the compound represented by formula I according to any one of claims 1 to 9, and one or more pharmaceutically acceptable carriers or excipients.

12. A method for preparing a pharmaceutical composition, the method comprising a step of mixing the crystal form of the compound represented by formula I according to any one of claims 1 to 9 with one or more pharmaceutically acceptable carriers or excipients.

13. Use of the crystal form of the compound represented by formula I according to any one of claims 1 to 9, or the pharmaceutical composition according to claim 11 in the manufacture of a medicament for the treatment of a physiological disorder, condition or disease in a patient, wherein the physiological disorder, condition or disease is preferably a respiratory disease, cough, inflammatory disease, skin disorder, ophthalmic disorder, depression, anxiety, phobia, bipolar disorder, alcohol dependence, neuroactive substance abuse, epilepsy, nociception, psychosis, schizophrenia, Alzheimer's disease, AIDS-related dementia, Towne's disease, stress-related disorder, obsessive/compulsive disorder, bulemia, anorexia nervosa, binge eating, mania, premenstrual syndrome, gastrointestinal dysfunction, atherosclerosis, fibrotic disorder, obesity, type II diabetes, headache, neuropathic pain, post-exercise pain, chronic pain syndrome, bladder disorder, urogenital disorder or vomiting or nausea.

14. A method for purifying a compound represented by formula I, the method comprising: mixing the compound represented by formula I with methyl tert-butyl ether under stirring, and separating the solid.

15. The method according to claim 14, the method further comprising mixing the separated solid with methyl tert-butyl ether and a second solvent under stirring, precipitating the solid and separating same, wherein the second solvent is selected from one or more of N,N-dimethylformamide, 1-methyl-2-pyrrolidone, dimethyl sulfoxide, tetrahydrofuran, ethyl acetate, isopropyl acetate, butyl acetate, dioxane, toluene, diethyl ether, isopropyl ether, methyl tert-butyl ether, dichloromethane, chloroform, acetone, acetonitrile, methanol, ethanol and isopropanol, preferably ethyl acetate.
